# EUROPÄISCHE PATENTSCHRIFT

(11) **EP 0 653 964 B1**
(45) Veröffentlichungstag und Bekanntmachung des Hinweises auf die Patenterteilung: **28.05.1997**
(21) Anmeldenummer: 93915691.5
(22) Anmeldetag: 04.08.1993
(51) Int. Cl.: B08B 9/46, G01N 1/26

(54) **VERFAHREN UND VORRICHTUNG ZUR INSPEKTION VON TRANSPORTIERTEN BEHÄLTERN**
METHOD AND DEVICE FOR THE INSPECTION OF CONTAINERS ON A CONVEYOR
PROCEDE ET DISPOSITIF POUR L'INSPECTION DE RECIPIENTS TRANSPORTES PAR UN CONVOYEUR

(30) Priorität: 06.08.1992 DE 4225984
(43) Veröffentlichungstag der Anmeldung: 24.05.1995
(73) Patentinhaber: KHS Maschinen- und Anlagenbau Aktiengesellschaft, D-44143 Dortmund (DE)
(72) Erfinder: STRAUCHMANN, Rüdiger, D-24943 Flensburg (DE); PETERS, Marten, D-24939 Flensburg (DE); GAISBAUER, Hubert, D-58710 Menden (DE)
(86) Internationale Anmeldenummer: DE9300692
(87) Internationale Veröffentlichungsnummer: WO9403287

(56) Entgegenhaltungen:
- EP-A- 0 306 307
- DE-U- 9 114 357
- US-A- 3 266 292
- US-A- 4 208 372
- US-A- 4 791 820

## Beschreibung

Die Erfindung bezieht sich auf ein Verfahren und die dazu geeignete Vorrichtung zur Entnahme von Luftproben aus Behältern und zu deren Zuleitung zu einer Analysestation.
Derartige Verfahren und Vorrichtungen sind aus der US-A 48 58 767 oder der DE 38 21 604 A1 entnehmbar.
Es ist bekannt, zur Messung von Verunreinigungen in Behältern eine Analyse der Behälteratmosphäre durch Probeentnahme und Probenzuführung zu einer direkt angeschlossenen automatisch arbeitenden Analysestation durchzuführen. Hierbei werden mittels Schlauch oder Rohranordnungen Volumenproben aus dem Behälter entnommen und der Analysestation zugeführt. Insbesondere bei der Überprüfung von wiederverwendbaren Kunststoffflaschen, die in hoher Anzahl anfallen, ist man dazu übergegangen, die jeweils zu untersuchenden Flaschen in einer geschlossenen Reihe einer rotierenden Maschine zuzuführen, die eine Vielzahl von Probeentnahmerohren am Rotorumfang über den Standplätzen der Flaschen aufweist. Die Rohre werden nach Aufnahme der Flaschen in diese abgesenkt, worauf ein entsprechender Teilstrom der darin befindlichen Luft angesaugt und zur Analysestation weitergeleitet wird. Es ist dabei erforderlich, daß die Flaschen zunächst auf ebenfalls an dem Rotor angeordneten Hubvorrichtungen aufgesetzt, dann gegen das Rohr angehoben und anschließend wieder abgeleitet und aus der Maschine abgeführt werden. Ebenfalls bekannt sind auch Systeme, bei denen die Flaschen auf am Rotorträger angeordneten Standflächen verbleiben und die Prüf- oder Saugrohre in entsprechend umgekehrter Weise in die Flasche abgesenkt werden. Der hierzu erforderliche maschinelle Aufwand, insbesondere für das Ein- und Ausfahren des Probeentnahmerohres, ist entsprechend hoch. Aufgrund der erforderlichen Hubbewegung sind auch die Durchlaufzeiten entsprechend lang.

Aus der EP-A-0306307 und der US-A-3266292 sind Vorrichtungen bekannt, mit denen ausgetriebene Behälterluft einzelnen Analysestationen direkt zugeleitet wird.

Die DE-U-9114357 zeigt eine Vorrichtung zur Fremdstoffinspektion von Gefäßen bei welcher die Meßsonden in die zu prüfenden Flaschen einführbar sind, wobei das Analysegas über Drehschieber der Analysevorrichtung zugeleitet wird.

Der Erfindung liegt die Aufgabe zugrunde, hier eine wesentliche Vereinfachung zu schaffen, mit welcher mit hoher Geschwindigkeit Proben aus dem Innenraum solcher Behältern entnommen werden können und eine schnelle und automatische Überprüfung der Behälter mit Hilfe einer stationären Gasentnahmevorrichtung möglich ist.

Diese Aufgabe wird gemäß der Erfindung bei einem Verfahren der eingangs genannten Art dadurch gelöst, daß die ausgetriebene Behälterluft oberhalb der Behälteröffnung innerhalb eines Sammelraumes bereitgehalten und zum Zwecke der Analyse aus diesem Raum abgesaugt wird.

Die zur Durchführung des Verfahrens geeignete Vorrichtung zeichnet sich dadurch aus, daß im Bereich einer vorbeigeführten Behälteröffnung eine mit Druckimpulsen eines Druckmediums beaufschlagbare Einblasdüse angeordnet und der Saugvorrichtung so zugeordnet ist, daß die durch die eingegebenen Druckimpulse ausgetriebene Behälterluft aufgenommen und der Analysestation zugeleitet werden kann und die Saugvorrichtung aus einem Luftspeicherbehälter besteht, an dem die zur Analysestation führende Saugleitung angeschlossen ist.

Mit diesen Verfahren und der dazu vorgeschlagenen Vorrichtung können kontinuierlich transportierte Behälter in hoher Stückzahl überprüft werden, ohne daß ein direktes oder tiefes Einführen eines Saugrohres in den Behälterinnenraum erforderlich wird. Durch die stationär angeordnete Analysestation und die entsprechenden Saugeinrichtungen oberhalb der Bewegungsebene der Behälter können diese unter der Station vorbeigeführt und dabei automatisch überprüft werden.

Im nachfolgenden wird die Erfindung anhand von in den Zeichnungen dargestellten Auführungsbeispielen näher erläutert.

Gemäß dem in Fig. 1 und Fig. 2 dargestellten Ausführungsbeispiel besteht die Vorrichtung aus einer Transportvorrichtung 1, auf der die zu überprüfenden Behälter 2 kontinuierlich und/oder diskontinuierlich transportiert werden. Oberhalb dieses Transporteurs befindet sich eine Saugvorrichtung 3. Diese besteht zweckmäßig aus einem düsenförmigen Einblasrohr 4, welches geringfügig oberhalb der Bewegungsebene der Behältermündungen 5 endet. Diese steht mit einem rohrförmigen Körper 6 in Verbindung, der zweckmäßig etwa in der Mitte eine Ansaugleitung 7 für die angesammelte und zu überprüfende Luft aufweist. Der rohrförmige Körper 6 ist beispielsweise gemäß Fig. 3 geringfügig mit seiner unteren Kante 8 oberhalb der Unterkante 9 der Einblasdüse 4 angeordnet. Besonders zweckmäßig ist dabei eine seitlich versetzte Anordnung der Einblasdüse 4 zum Zentrum des Körpers 6.

Der auf der Transportvorrichtung 1 transportierte Behälter 2 wird kontinuierlich oder diskontinuierlich unterhalb der Saugvorrichtung 3 vorbei transportiert, wobei durch nicht weiter dargestellte Impulsgeber durch die Einblasdüse ein Luftstoß in die Behälter eingegeben wird. Dieser veranlaßt unmittelbar den Austritt der darin befindlichen Atmosphäre, die dann in den rohrförmigen Körper 6 gelangt und durch ein beispielsweise ebenfalls taktweise schaltbares Analyserohr 7 zu der nicht dargestellten Analysevorrichtung weitergeleitet wird. Die in dem rohrförmigen Körper 6 verbleibende Restluft kann durch seitliche oder stirnseitige Öffnungen 10 relativ schnell entweichen. Es ist aber auch denkbar, hierfür zusätzlich eine entsprechende Ausleitmaßnahme vorzusehen.

Gemäß Fig. 4 besteht die Saugvorrichtung 3 aus einem langgestreckten Einblasrohr 4', welches über eine längere Wegstrecke der Behälter reicht. An einer Stelle dieser Düse ist der rohrförmige Körper 6 angeordnet. Zweckmäßig befindet sich die langgestreckte Düse 4' am Rand der Behältermündung, wodurch ein relativ großer Austrittsbereich für die auszutreibende Luft gebildet wird. Zweckmäßig wird dabei die Düse so gerichtet, daß die Luft entlang der Innenwandung vorbeistreicht und dadurch eine relativ große Restöffnung für die auszuströmende bzw. die herausgedrückte Luft verbleibt.

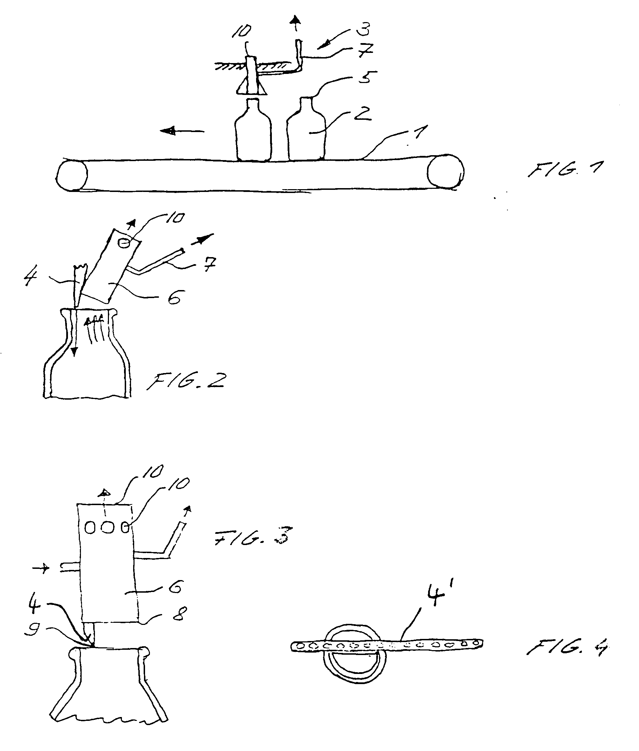

Fig. 3 zeigt ebenfalls eine am Rand der Behältermündung angeordnete Düse 4, wobei sich die Behälter in die Zeichnungsebene hineinbewegen und aufgrund dieser Anordnung ebenfalls ein großer Eintrittsquerschnitt für die aufzunehmende Luft aus dem Behälter an den rohrförmigen Körper 6 verbleibt. Die eingesetzten Düsen 4 können bestimmte Ausgestaltungen haben, die eine besonders günstige Strömungsführung innerhalb der Flasche gestatten. Auf diese Weise kann die auszutreibende Behälterluft relativ problemlos entweichen und zur Analyse zugeführt werden. Bei einem taktweisen Arbeiten ist es auch denkbar, die Saugvorrichtung ohne eine Blasdüse einzusetzen und jeweils unmittelbar bei einem darunter befindlichen Behälter durch erhöhten Unterdruck einzuschalten, wobei zweckmäßig noch eine Umgebungsglocke vorgesehen ist, die eine gewisse Abschirmung zur Umgebungsluft gestattet.

Es ist auch denkbar, die einzelnen Saugvorrichtungen innerhalb der stationären Gasentnahmevorrichtung über dem Flaschenstrom rotieren zu lassen, wobei dieser in einer kreisförmigen Bewegungsbahn unterhalb der jeweils zugeordneten Saugvorrichtung 3 vorbeiläuft. Im weiteren Rotationsabschnitt der Saugvorrichtungen 3 kann dann eine entsprechende Ableitung des Gases zum Analysegerät durchgeführt werden.

Die keine einwandfreie Atmosphäre beinhaltenden Behälter 2 können dann über bekannte Ausleitsysteme ausgeschieden werden.

## Patentansprüche

1. Verfahren zur Entnahme von Luftproben aus Behältern (2) und zu deren Zuleitung zu einer Analysestation, wobei mit einer im Bereich einer Behälteröffnung angeordneten Einblasdüse (4) Druckimpulse eines Druckmediums in die Behälter (2) eingegeben und die dadurch ausgetriebene Behälterluft aufgefangen wird und bei dem die Behälter auf einer Transportvorrichtung an einer stationären Gasentnahmevorrichtung vorbeigeführt werden, mit welcher jedem vorbeigeführten Behälter (2) ein Teil der darin enthaltenen Luft berührungslos entnehmbar und der Analysestation zuleitbar ist und jedem Behälter (2) ein oder mehrere Druckimpulse eines Druckmediums zum Austreiben der Behälterluft eingegeben und die dadurch ausgetriebene Behälterluft der Analysestation zugeleitet wird, ***dadurch gekennzeichnet***, daß die ausgetriebene Behälterluft oberhalb der Behälteröffnung innerhalb eines Sammelraumes (Körper 6) bereitgehalten und zum Zwecke der Analyse aus diesem Raum abgesaugt wird.

2. Verfahren nach Anspruch 1, ***dadurch gekennzeichnet***, daß die stationäre Gasentnahmevorrichtung durch Ansaugen wirksam ist.

3. Vorrichtung zur Entnahme von Luftproben aus Behältern (2) und zu deren Zuleitung zu einer Analysestation mit einer zu dieser führenden Saugleitung (7), die in einer im Bereich der Behälter angebrachten Saugvorrichtung endet, und mit einer die Behälter daran vorbeiführenden Transportvorrichtung, ***dadurch gekennzeichnet***, daß im Bereich einer vorbeigeführten Behälteröffnung eine mit Druckimpulsen eines Druckmediums beaufschlagbare Einblasdüse (4) angeordnet und der Saugvorrichtung so zugeordnet ist, daß die durch die eingegebenen Druckimpulse ausgetriebene Behälterluft aufgenommen und der Analysestation zugeleitet werden kann und die Saugvorrichtung aus einem Luftspeicherbehälter (Körper 6) besteht, an dem die zur Analysestation führende Saugleitung (7) angeschlossen ist.

4. Vorrichtung nach Anspruch 3, ***dadurch gekennzeichnet***, daß die Saugvorrichtung aus einem rohrförmigen Körper (6) gebildet ist, der an seinem Mantel Gasaustauschöffnungen (10) aufweist.

5. Vorrichtung nach einem der Ansprüche 3 oder 4, ***dadurch gekennzeichnet***, daß die eine oder mehrere Einblasdüsen (4) asymetrisch zur Behälterachse und zur Transportrichtung der Behälter (2) vorgesehen sind und die Saugvorrichtung über dem Hauptausströmquerschnitt der Behälteröffnung angeordnet ist.

## Claims

1. Method for taking air samples from containers (2) and feeding them to an analytical device; comprising an injection nozzle (4) positioned in the area of a container opening and transferring pressure impulses of a pressure medium into the containers (2), by this absorbing the expelled air, by means of which the containers are conducted on a conveyor past a stationary analytical device, with which part of the air contained in each of the containers (2) passing the device can be extracted without contact and fed to the analytical device, and one or several pressure impulses of a pressure medium are transmitted to each container (2) for expelling the container air; and the air expelled by this is fed to the analytical device, ***characterised in that*** the expelled air is kept in readiness in a collection chamber (body 6) above the container opening and sucked out of this chamber for analysis.

2. Method according to claim 1, ***characterised in that*** the stationary analytical device becomes effective by absorption.

3. Device for taking air samples from containers (2) and feeding them to an analytical device with a suction pipe (7) leading to the analytical device and ending in a suction device placed in the area of the containers and with a conveyor conducting the containers past the suction device, ***characterised in that*** an injection nozzle (4) reacting to pressure impulses of a pressure medium is positioned in the area of the passing container opening and placed in such a position to the suction device that the air expelled by the applied pressure impulses is absorbed and fed to the analytical device, and the suction device consists of an air cell container (body 6) which has a connection with the suction pipe (7) leading to the analytical device.

4. Device according to claim 3, ***characterised in that*** the suction device is formed by a tubular body (6) providing escape openings (10) on its wall.

5. Device according to one of the preceding claims 3 or 4, ***characterised in that*** one or several injection nozzles (4) are positioned asymmetrically about the container axis and the transport direction of the containers (2) and the suction device is arranged above the escape cross-section of the container opening.

## Revendications

1. Procédé de prélèvement d'échantillons d'air dans des récipients (2) et d'envoi de ceux-ci vers un poste d'analyse, dans lequel au moyen d'une buse de soufflage (4), placée dans la zone d'une ouverture du récipient, des impulsions de pression d'un fluide sous pression sont introduites dans les récipients (2) et l'air des récipients, ainsi chassé, est recueilli, et dans lequel les récipients sont transportés, sur un dispositif de transport, devant un dispositif de prélèvement de gaz fixe, au moyen duquel une partie de l'air contenu dans chaque récipient (2) passant devant le dispositif de prélèvement peut être prélevée sans contact et acheminée vers le poste d'analyse, et à chaque récipient (2) sont imparties une ou plusieurs impulsions de pression d'un fluide sous pression, en vue de chasser l'air du récipient, et l'air du récipient ainsi chassé est acheminé vers le poste d'analyse, caractérisé en ce que l'air chassé du récipient est tenu à disposition, au-dessus de l'ouverture du récipient, à l'intérieur d'une enceinte de collecte (corps 6) et est aspiré de cette enceinte, en vue de l'analyse.

2. Procédé selon la revendication 1, caractérisé en ce que le dispositif de prélèvement de gaz fixe agit par aspiration.

3. Dispositif de prélèvement d'échantillons d'air dans des récipients (2) et d'acheminement de ceux-ci vers un poste d'analyse, comprenant une conduite d'aspiration (7), menant à celui-ci, qui se termine dans un dispositif d'aspiration, placé dans la zone des récipients, et comprenant un dispositif de transport, faisant passer les récipients devant le dispositif d'aspiration, caractérisé en ce que dans la zone de passage d'une ouverture de récipient, est placée une buse de soufflage (4), soumise à des impulsions de pression d'un fluide sous pression, et laquelle est associée au dispositif d'aspiration, de manière que l'air des récipients, chassé par les impulsions de pression introduites, peut être recueilli et acheminé vers le poste d'analyse et le dispositif d'aspiration est constitué d'un réservoir de stockage d'air (corps 6), auquel est raccordée la conduite d'aspiration (7), menant au poste d'analyse.

4. Dispositif selon la revendication 3, caractérisé en ce que le dispositif d'aspiration est formé par un corps tubulaire (6), qui présente des ouvertures d'échange de gaz (10) sur son enveloppe.

5. Dispositif selon l'une des revendications 3 ou 4, caractérisé en ce qu'une ou plusieurs buses de soufflage (4) sont prévues asymétriquement par rapport à l'axe des récipients et par rapport au dispositif de transport des récipients (2) et le dispositif d'aspiration est placé au-dessus de la section transversale d'écoulement principale de l'ouverture des récipients.
